# EUROPEAN PATENT SPECIFICATION

(11) **EP 4 295 813 B1**
(45) Date of publication and mention of the grant of the patent: **18.11.2026**
(21) Application number: 22756432.5
(22) Date of filing: 11.02.2022
(51) Int. Cl.: A61F 2/12, A61L 27/18, A61L 27/52

(54) **BREAST IMPLANT COMPRISING SILICONE GELS OF VARIOUS PROPERTIES**
BRUSTIMPLANTAT MIT SILIKONGELEN VERSCHIEDENER EIGENSCHAFTEN
IMPLANT MAMMAIRE COMPRENANT DES GELS DE SILICONE PRÉSENTANT DIVERSES PROPRIÉTÉS

(30) Priority: 19.02.2021 KR 20210022731
(43) Date of publication of application: 27.12.2023
(73) Proprietor: Osstemimplant Co., Ltd., Gangseo-gu Seoul 07789 (KR)
(72) Inventor: SIM, Eun Jung, Seoul 07568 (KR); KIM, Byung Hwi, Yongin-si, Gyeonggi-do 16968 (KR); KIM, Min Kyoung, Incheon 22765 (KR); SONG, Ju Dong, Busan 48272 (KR); JANG, Il Seok, Yangsan-si, Gyeongsangnam-do 50653 (KR)
(74) Representative: Beck & Rössig European Patent Attorneys
(86) International application number: PCT/KR2022/002065
(87) International publication number: WO 2022/177233

(56) References cited:
- WO-A1-2007/050693
- JP-A- 2009 513 261
- KR-A- 20120 123 093
- KR-A- 20120 123 093
- KR-A- 20130 004 575
- US-A- 3 681 787
- US-A- 4 676 795
- US-A- 4 701 230
- US-A- 4 773 909
- US-A1- 2019 083 234

## Description

The present invention relates to a breast implant including two or more types of silicone gels having different cohesive strengths.

Silicone gel breast implants are manufactured by filling a silicone gel having appropriate viscosity in a silicone shell forming the outer shell of the implant and have excellent durability and tactile sensation compared to saline breast implants. The silicone gel breast implant is a Class IV medical device and absolutely requires special biocompatibility, safety, and stability because it is generally present for a long period of time after being implanted in vivo. Also, in the case of a breast implant, a natural shape or a texture similar to skin tissue need to be realized. Therefore, it has been developed to secure properties required as a medical device by adjusting the thickness of a silicone shell or changing a filler component and simultaneously have a natural shape, but there are other side effects on the shape

A rippling phenomenon (rippling deformity) refers to a folding phenomenon of an implant shell and means a phenomenon in which wavy wrinkles are formed on an implant and revealed through the skin. This phenomenon is one of the side effects that frequently occur in use of silicone gel breast implants and may lead to a reoperation. The major cause of rippling is the downward tilting of a gel, and when the implant is present in an upright state for a long period of time after being implanted in vivo, the silicone gel filled in the implant tilts downward due to gravity, and thus wrinkles are formed in the area filled with a relatively small amount of silicone gel. Therefore, the rippling phenomenon continuously occurs at a specific site of the implant, and the properties of the shell folded at the corresponding site are degraded, and thus the implant may subsequently rupture in vivo due to an impact.

When the silicone gel breast implant ruptures, the silicone gel filled in the implant may leak out. The long-term leakage of the silicone gel may change the volume and shape of the implant, and the leaked silicone gel may adversely affect internal tissues or organs, leading to severe side effects such as complications, tissue necrosis, and the like.

A breast implant comprising the features of the preamble portion of either of claims 1 and 2 is known from WO 2007/050693 A1.

A breast implant is also vaguely mentioned in US 2019/083234 A.

The present invention is directed to providing a silicone gel breast implant that on the one hand reduces or avoids the rippling phenomenon and on the other hand provides high safety standards.

This technical problem is solved by a breast implant comprising all features of either claim 1 or claim 2. Since a breast implant includes two or more types of silicone gels having different cohesive strengths, a rippling phenomenon, which is a side effect that occurs in a silicone gel breast implant, is prevented, and thus the risk of in vivo rupture can be reduced.

However, it is to be understood that the effect of an aspect of the specification is not limited to the above-described effect but include all effects deducible from the configuration described in the detailed description of the specification or in the claims.In the drawings:
FIG. 1 shows a longitudinal sectional view of a breast implant having reinforced gel cohesion in a side portion according to a first embodiment of the present invention.
FIG. 2 shows a transverse sectional view of the breast implant having reinforced gel cohesion in a side portion of the first embodiment.
FIG. 3 shows a longitudinal sectional view of a breast implant having reinforced gel cohesion in a lower portion according to a second embodiment of the present invention.
FIG. 4 shows a transverse sectional view of a lower portion of a breast implant having reinforced gel cohesion in a lower portion according to the second embodiment .

Hereinafter, the present invention will be described with reference to the accompanying drawings. However, it should be understood that the descriptions of the specification can be implemented in various other forms. Also, in the drawings, descriptions of parts unrelated to the detailed description are omitted to clearly describe an aspect of the specification. Throughout the specification, like numbers refer to like elements.

Throughout the specification, the phrase a certain part is "connected" to another part means that the certain part is not only "directly connected" to the other part but also "indirectly connected" to the other part through another member interposed between the two parts. Also, the phrase a certain part "includes" a certain element means that the certain part may further include, instead of excluding, another element unless particularly indicated otherwise.

When a numerical value is presented in the specification, the value has the precision of significant digits provided in accordance with the standard rules in chemistry for significant digits unless its specific range is stated otherwise. For example, the numerical value 10 includes the range of 5.0 to 14.9 and the numerical value 10.0 includes the range of 9.50 to 10.49.

Hereinafter, an embodiment of the present invention will be described in detail with reference to the accompanying drawings.

### Breast implant including silicone gels having different cohesive strengths

A breast implant according to includes: a filler including two or more types of silicone gels having different cohesive strengths; and a silicone shell surrounding the filler.

The cross-section of the breast implant may have one shape selected from a circular shape, an oval shape, and a teardrop shape. Generally, since users have different breast shapes, the type of the breast implant may be selected in consideration of an original shape and preference of users such as a desired shape, a desired size, and the like.

The silicone gel may be polyorganosiloxane. The polyorganosiloxane may be one or more selected from the group consisting of dimethylsiloxane, methyl hydrogen siloxane, methylphenylsiloxane, diphenylsiloxane, dimethylvinylsiloxane, and trifluoropropylsiloxane, but the type thereof is not particularly limited as long as it can realize the functional characteristics of a breast implant.

The two or more types of silicone gels may have different properties such as viscosity, elasticity, flowability, and the like.

Since the breast implant includes two or more types of silicone gels having different cohesive strengths, the degree at which the gel tilts downward can be adjusted, the resistance of a site where folding occurs in the implant can be increased to prevent a rippling phenomenon, and the stability of the implant can be enhanced. In the case of a conventional breast implant including a silicone gel having a single property, a rippling phenomenon frequently occurs due to the downward tilting of a gel and becomes a major cause of implant rupture.

The silicone shell may be manufactured by applying a silicone polymer dispersion onto a mandrel having a desired shape and performing curing. The silicone polymer dispersion may form an elastomer coating on the mandrel, and the coating may be cured, and the solvent may be evaporated.

An inside of the silicone shell may be divided into two or more areas, and the two or more types of silicone gels having different cohesive strengths may be disposed in the two or more areas, respectively. For example, the two or more areas may be divided by an interface formed between the two or more types of silicone gels having different cohesive strengths.

The inside of the silicone shell may be divided into an area where a rippling phenomenon occurs and an area where a rippling phenomenon does not occur, and the silicone gel disposed in the area where a rippling phenomenon occurs may have higher cohesive strength, higher viscosity, or lower flowability than the silicone gel disposed in the area where a rippling phenomenon does not occur.

Since a silicone gel having high cohesive strength, high viscosity, or low flowability is disposed in the area where a rippling phenomenon occurs in the inside of the silicone shell, the breast implant according to an aspect of the specification can prevent a rippling phenomenon and increase rupture resistance to enhance the stability of the implant.

### Breast implant having reinforced gel cohesion in side portion (first embodiment)

In the breast implant according to a first embodiment, the inside of the silicone shell may be divided into a side portion 12 and a middle portion 13, and two types of silicone gels having different cohesive strengths may be disposed in the side portion 12 and the middle portion 13, respectively.

The side portion 12 may be formed along a side circumference of the breast implant, and the middle portion 13 may refer to all areas except for the side portion 12 in the inside of the silicone shell.

The silicone gel disposed in the side portion 12 has higher cohesive strength than the silicone gel disposed in the middle portion 13.

The silicone gel disposed in the side portion 12 has higher viscosity and lower flowability than the silicone gel disposed in the middle portion 13.

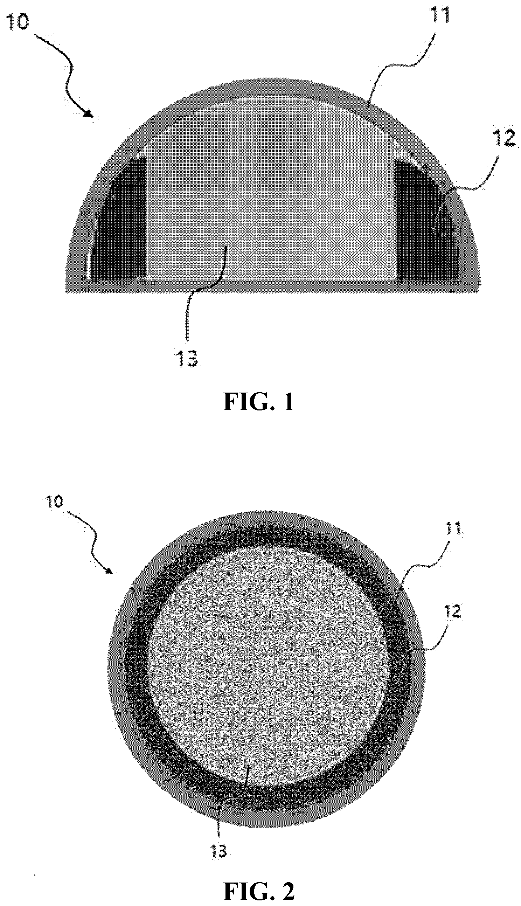

FIGS. 1 and 2 show the longitudinal sectional view and transverse sectional view of a breast implant having reinforced gel cohesion in a side portion, respectively.

Referring to FIGS. 1 and 2, a breast implant 10 having reinforced gel cohesion in a side portion includes: a silicone shell 11; a side portion 12 filled with a cohesive strength-reinforced silicone gel; and a middle portion 13 filled with a general silicone gel.

Since the side portion 12 is filled with a cohesive strength-reinforced silicone gel having high cohesive strength compared to the middle portion 13, the degree at which the gel tilts downward can be adjusted, the resistance of a site where folding occurs in the implant can be increased to prevent a rippling phenomenon, and the stability of the implant can be enhanced.

Since the middle portion 13 is filled with a general silicone gel having lower cohesive strength than the cohesive strength-reinforced silicone gel filled in the side portion 12, the breast implant can realize a natural shape and a texture similar to skin tissue.

The silicone gel disposed in the side portion 12 has a cohesive strength of 23 to 40 N. For example, the silicone gel disposed in the side portion 12 may have a cohesive strength of 23 N, 24 N, 25 N, 26 N, 27 N, 28 N, 29 N, 30 N, 31 N, 32 N, 33 N, 34 N, 35 N, 36 N, 37 N, 38 N, 39 N, 40 N, or a cohesive strength in a range between two of the above values. When the silicone gel disposed in the side portion 12 has a cohesive strength less than 10 N, a rippling phenomenon occurs, and thus the possibility of implant rupture may increase, and when the silicone gel disposed in the side portion 12 has a cohesive strength exceeding 40 N, a natural shape or a texture similar to skin tissue may not be realized.

The side portion 12 occupies 20 to 50 vol% based on the total volume of the inside of the silicone shell 11. For example, the volume of the side portion 12 may be 20 vol%, 25 vol%, 30 vol%, 35 vol%, 40 vol%, 45 vol%, 50 vol%, or in a range between two of the above values based on the total volume of the inside of the silicone shell 11. When the volume of the side portion 12 is less than 10 vol% based on the total volume of the inside of the silicone shell 11, a rippling phenomenon occurs, and thus the possibility of implant rupture may increase, and when the volume of the side portion 12 exceeds 50 vol%, a natural shape or a texture similar to skin tissue may not be realized.

### Breast implant having reinforced gel cohesion in lower portion (second embodiment)

In the breast implant according to a second embodiment, the inside of the silicone shell is divided into an upper portion 23 and a lower portion 22, and two types of silicone gels having different cohesive strengths may be disposed in the upper portion 23 and the lower portion 22, respectively.

The upper portion 23 may refer to an area located at the front when the breast implant is implanted in vivo, and the lower portion 22 may refer to an area located at the rear when the breast implant is implanted in vivo.

The silicone gel disposed in the lower portion 22 has higher cohesive strength than the silicone gel disposed in the upper portion 23.

The silicone gel disposed in the lower portion 22 has higher viscosity and lower flowability than the silicone gel disposed in the upper portion 23.

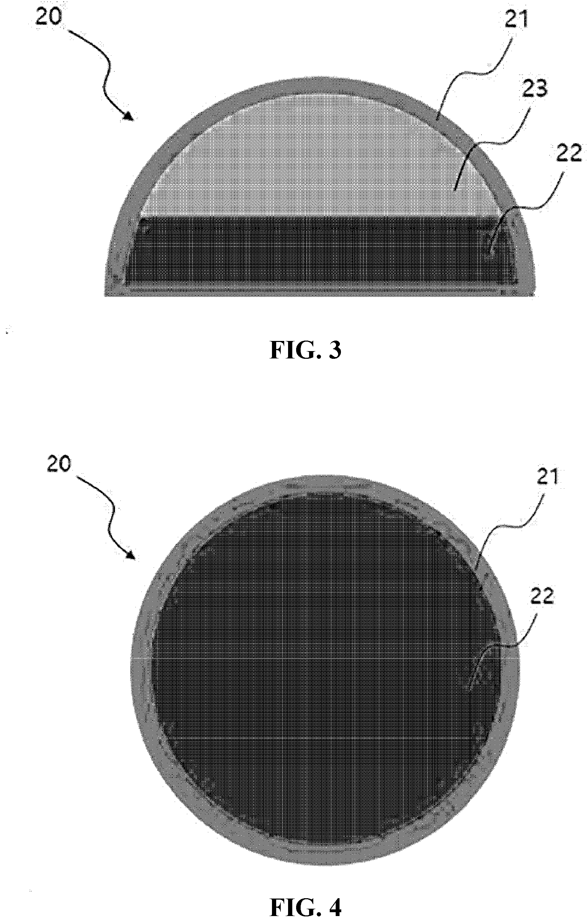

FIGS. 3 and 4 show the longitudinal sectional view of a breast implant having reinforced gel cohesion in a lower portion according to the second embodiment and the transverse sectional view of the lower portion of the breast implant, respectively.

Referring to FIGS. 3 and 4, a breast implant 20 having reinforced gel cohesion in a lower portion includes: a silicone shell 21; a lower portion 22 filled with a cohesive strength-reinforced silicone gel; and an upper portion 23 filled with a general silicone gel.

Since the lower portion 22 is filled with a cohesive strength-reinforced silicone gel having high cohesive strength compared to the upper portion 23, the degree at which the gel tilts downward can be adjusted, the resistance of a site where folding occurs in the implant can be increased to prevent a rippling phenomenon, and the stability of the implant can be enhanced.

Since the upper portion 23 is filled with a general silicone gel having lower cohesive strength than the cohesive strength-reinforced silicone gel filled in the lower portion 22, the breast implant can realize a natural shape and a texture similar to skin tissue.

The silicone gel disposed in the lower portion 22 has a cohesive strength of 23 to 40 N. For example, the silicone gel disposed in the lower portion 22 may have a cohesive strength of 23 N, 24 N, 25 N, 26 N, 27 N, 28 N, 29 N, 30 N, 31 N, 32 N, 33 N, 34 N, 35 N, 36 N, 37 N, 38 N, 39 N, 40 N, or a cohesive strength in a range between two of the above values. When the silicone gel disposed in the lower portion 22 has a cohesive strength less than 10 N, a rippling phenomenon occurs, and thus the possibility of implant rupture may increase, and when the silicone gel disposed in the lower portion 22 has a cohesive strength exceeding 40 N, a natural shape or a texture similar to skin tissue may not be realized.

The lower portion 22 occupies 20 to 50 vol% based on the total volume of the inside of the silicone shell 21. For example, the volume of the lower portion 22 may be 20 vol%, 25 vol%, 30 vol%, 35 vol%, 40 vol%, 45 vol%, 50 vol%, or in a range between two of the above values based on the total volume of the inside of the silicone shell 21. When the volume of the lower portion 22 is less than 10 vol% based on the total volume of the inside of the silicone shell 21, a rippling phenomenon occurs, and thus the possibility of implant rupture may increase, and when the volume of the lower portion 22 exceeds 50 vol%, a natural shape or a texture similar to skin tissue may not be realized.

Hereinafter, examples of the specification will be described in further detail. However, the following experimental results are obtained from only a few selected examples of the invention, and the scope and contents of the specification should not be interpreted as being reduced or limited by the few selected examples. The effects of each of the various embodiments of the specification, which are not explicitly set forth below, will be described in detail in relevant sections.

### Example 1: Breast implant having reinforced gel cohesion in side portion

A side portion 12 occupying 20 vol% based on the total volume of an inside of a silicone shell 11 was filled with a cohesive strength-reinforced silicone gel having a cohesive strength of 23 N, and a middle portion 13 was filled with a general silicone gel having a cohesive strength of 13 N, thereby manufacturing a breast implant 10 having reinforced gel cohesion in the side portion.

### Example 2: Breast implant having reinforced gel cohesion in lower portion

An upper portion 23 was filled with a general silicone gel having a cohesive strength of 13 N, and a lower portion 22 occupying 30 vol% based on the total volume of an inside of a silicone shell 21 was filled with a cohesive strength-reinforced silicone gel having a cohesive strength of 26 N, thereby manufacturing a breast implant 20 having reinforced gel cohesion in the lower portion.

### Comparative Example 1

A breast implant 10 having reinforced gel cohesion in a side portion was manufactured in the same manner as in Example 1, except that the cohesive strength-reinforced silicone gel filled in a side portion 12 had a cohesive strength of 15 N.

### Comparative Example 2

A breast implant 10 having reinforced gel cohesion in a side portion was manufactured in the same manner as in Example 1, except that the volume of a side portion 12 occupied 5 vol% based on the total volume of an inside of a silicone shell 11.

### Comparative Example 3

A breast implant 20 having reinforced gel cohesion in a lower portion was manufactured in the same manner as in Example 2, except that the cohesive strength-reinforced silicone gel filled in a lower portion 22 had a cohesive strength of 15 N.

### Comparative Example 4

A breast implant 20 having reinforced gel cohesion in a lower portion was manufactured in the same manner as in Example 2, except that the volume of a lower portion 22 occupied 10 vol% based on the total volume of an inside of a silicone shell 21.

### Comparative Example 5: Breast implant including silicone gel having single property

A general silicone gel having a cohesive strength of 13 N was filled in the entire inside of the silicone shell to manufacture a breast implant including a silicone gel having a single property.

The cohesive strength of each gel in the examples and comparative examples is a value obtained by measuring the pressure at the time the silicone gel discharged from a 1-mm circular hole is separated when negative pressure is applied after the hole is made in the outside of the implant.

### Experimental Example 1: Observation of shape change

In order to confirm the effect of the breast implant including silicone gels having various properties according to an aspect of the specification on prevention of a rippling phenomenon, a change in shape of each implant according to Examples 1 and 2 and Comparative Examples 1 to 5 when one side of the implant was held and lifted was observed.

As a result, in the case of the implant of Comparative Example 2 in which the volume filled with a cohesive strength-reinforced silicone gel occupied less than 10 vol% based on the total volume of the inside of the silicone shell 21 and the implant of Comparative Example 5 in which a silicone gel filled therein had a single property, the downward tilting of the gel due to gravity and the rippling phenomenon were observed. On the other hand, in the case of the implants of Examples 1 and 2, the downward tilting of the gel due to gravity was significantly reduced, and rippling did not occur, as compared with the implants of Comparative Examples 1 to 5.

### Experimental Example 2: Evaluation of rupture safety and stability

In order to confirm the effect of the breast implant including silicone gels having various properties according to an aspect of the specification on reduction in the risk of rupture, fatigue was applied 6 million times to each implant of Examples 1 and 2 and Comparative Examples 1 to 5, and rupture safety and stability were evaluated.

Referring Table 1 below, in the case of the implant of Comparative Example 2 in which the volume filled with a cohesive strength-reinforced silicone gel occupied less than 10 vol% based on the total volume of the inside of the silicone shell 21 and the implant of Comparative Example 5 in which a silicone gel filled therein had a single property, when fatigue was applied 4 million times or more, the side portion ruptured, and the internal filler leaked. On the other hand, in the case of the implants of Examples 1 and 2, it was confirmed that, even when fatigue was applied 6 million times or more, rupture did not occur, and thus rupture safety and stability were excellent.

In the case of the implants of Comparative Examples 1 and 3 using a cohesive strength-reinforced silicone gel having a cohesive strength of 15 N and the implant of Comparative Example 4 in which the volume filled with a cohesive strength-reinforced silicone gel occupied 10 vol% based on the total volume of the inside of the silicone shell, when fatigue was applied 6 million times or more, rupture occurred. From this result, it was confirmed that rupture safety and stability were improved compared to the implants of Comparative Examples 2 and 5.

**[Table 1]**

| Classification | 2 million times | 4 million times | 6 million times |
|---|---|---|---|
| Example 1 | Nothing wrong | Nothing wrong | Nothing wrong |
| Example 2 | Nothing wrong | Nothing wrong | Nothing wrong |
| Comparative Example 1 | Nothing wrong | Nothing wrong | Rupture of side portion |
| Comparative Example 2 | Nothing wrong | Rupture of side portion | Rupture of side portion |
| Comparative Example 3 | Nothing wrong | Nothing wrong | Rupture of side portion |
| Comparative Example 4 | Nothing wrong | Nothing wrong | Rupture of side portion |
| Comparative Example 5 | Nothing wrong | Rupture of side portion | Rupture of side portion |

The foregoing description is intended for illustration, and it will be understood by those skilled in the art to which the invention pertains that the invention can be easily modified in other specific forms. For example, each of the constituents described as being one combined entity may be implemented separately, and similarly, constituents described as being separate entities may be implemented in a combined form.

### List of Reference Numerals

10: Breast implant having reinforced gel cohesion in side portion
11: Silicone shell
12: Side portion
13: Middle portion
20: Breast implant having reinforced gel cohesion in lower portion
21: Silicone shell
22: Lower portion
23: Upper portion

## Claims

1. A breast implant (10) comprising:
a filler including two or more types of silicone gels having different cohesive strengths; and
a silicone shell (11) surrounding the filler;
wherein the inside of the silicone shell is divided into a side portion (12) and a middle portion (13), and two types of silicone gels having different cohesive strengths are disposed in the side portion and the middle portion, respectively;
wherein the silicone gel disposed in the side portion (12) has higher cohesive strength than the silicone gel disposed in the middle portion (13);
**characterized in that**
the silicone gel disposed in the side portion has a cohesive strength of 23 to 40 N, wherein the cohesive strength is a value obtained by measuring the pressure at the time the silicone gel discharged from a 1-mm circular hole is separated when negative pressure is applied; and
the side portion occupies 20 to 50 vol% based on a total volume of the inside of the silicone shell.

2. A breast implant (20) comprising:
a filler including two or more types of silicone gels having different cohesive strengths; and
a silicone shell (21) surrounding the filler;
wherein the inside of the silicone shell (21) is divided into an upper portion (23) and a lower portion (22), and two types of silicone gels having different cohesive strengths are disposed in the upper portion and the lower portion, respectively; and
wherein the silicone gel disposed in the lower portion (22) has higher cohesive strength than the silicone gel disposed in the upper portion (23);
**characterized in that**
the silicone gel disposed in the lower portion (22) has a cohesive strength of 23 to 40 N, wherein the cohesive strength is a value obtained by measuring the pressure at the time the silicone gel discharged from a 1-mm circular hole is separated when negative pressure is applied; and
the lower portion occupies 20 to 50 vol% based on the total volume of the inside of the silicone shell (21).

## Patentansprüche

1. Brustimplantat (10), umfassend:
einen Füllstoff, der zwei oder mehr Arten von Silikongelen mit unterschiedlicher Kohäsionsfestigkeit aufweist; und
eine Silikonhülle (11), die den Füllstoff umgibt;
wobei der Innenraum der Silikonhülle in einen seitlichen Abschnitt (12) und einen mittleren Abschnitt (13) unterteilt ist und zwei Arten von Silikongelen mit unterschiedlicher Kohäsionsfestigkeit jeweils in dem seitlichen Abschnitt und dem mittleren Abschnitt angeordnet sind;
wobei das in dem seitlichen Abschnitt (12) angeordnete Silikongel eine höhere Kohäsionsfestigkeit als das in dem mittleren Abschnitt (13) angeordnete Silikongel aufweist;
**dadurch gekennzeichnet, dass**
das in dem seitlichen Abschnitt angeordnete Silikongel eine Kohäsionsfestigkeit von 23 bis 40 N aufweist, wobei die Kohäsionsfestigkeit ein Wert ist, der durch Messen des Drucks zu dem Zeitpunkt erhalten wird, zu dem sich das aus einer kreisförmigen Öffnung mit einem Durchmesser von 1 mm austretende Silikongel unter Einwirkung von Unterdruck ablöst; und
der seitliche Abschnitt 20 bis 50 Vol.-%, basierend auf einem Gesamtvolumen des Innenraums der Silikonhülle, einnimmt.

2. Brustimplantat (20), das umfassen:
einen Füllstoff, der zwei oder mehr Arten von Silikongelen mit unterschiedlicher Kohäsionsfestigkeit aufweist; und
eine Silikonhülle (21), die den Füllstoff umgibt;
wobei der Innenraum der Silikonhülle (21) in einen oberen Abschnitt (23) und einen unteren Abschnitt (22) unterteilt ist und zwei Arten von Silikongelen mit unterschiedlicher Kohäsionsfestigkeit jeweils in dem oberen Abschnitt und dem unteren Abschnitt angeordnet sind; und
wobei das in dem unteren Abschnitt (22) angeordnete Silikongel eine höhere Kohäsionsfestigkeit als das in dem oberen Abschnitt (23) angeordnete Silikongel aufweist;
**dadurch gekennzeichnet, dass**
das in dem unteren Abschnitt (22) angeordnete Silikongel eine Kohäsionsfestigkeit von 23 bis 40 N aufweist, wobei die Kohäsionsfestigkeit ein Wert ist, der durch Messen des Drucks zu dem Zeitpunkt erhalten wird, zu dem sich das aus einer kreisförmigen Öffnung mit einem Durchmesser von 1 mm austretende Silikongel unter Einwirkung von Unterdruck ablöst; und
der untere Abschnitt 20 bis 50 Vol.-%, basierend auf dem Gesamtvolumen des Innenraums der Silikonhülle (21), einnimmt.

## Revendications

1. Implant mammaire (10) comprenant:
un produit de remplissage comprenant deux ou plusieurs types de gels de silicone présentant des forces de cohésion différentes; et
une enveloppe en silicone (11) entourant le produit de remplissage;
dans lequel l'intérieur de l'enveloppe en silicone est divisé en une partie latérale (12) et une partie centrale (13), et deux types de gels de silicone présentant des forces de cohésion différentes sont disposés respectivement dans la partie latérale et la partie centrale;
dans lequel le gel de silicone disposé dans la partie latérale (12) présente une force de cohésion supérieure à celle du gel de silicone disposé dans la partie centrale (13);
**caractérisé en ce que**
le gel de silicone disposé dans la partie latérale présente une force de cohésion comprise entre 23 et 40 N, dans lequel la force de cohésion est une valeur obtenue en mesurant la pression au moment où le gel de silicone libéré à partir d'un orifice circulaire de 1 mm est séparé lorsqu'une pression négative est appliquée; et
la partie latérale occupe 20 à 50 % en volume par rapport au volume total de l'intérieur de l'enveloppe en silicone.

2. Implant mammaire (20) comprenant:
un produit de remplissage comprenant deux ou plusieurs types de gels de silicone présentant des forces de cohésion différentes; et
une enveloppe en silicone (21) entourant le produit de remplissage;
dans lequel l'intérieur de l'enveloppe en silicone (21) est divisé en une partie supérieure (23) et une partie inférieure (22), et deux types de gels de silicone présentant des forces de cohésion différentes sont disposés respectivement dans la partie supérieure et la partie inférieure; et
dans lequel le gel de silicone disposé dans la partie inférieure (22) présente une force de cohésion supérieure à celle du gel de silicone disposé dans la partie supérieure (23);
**caractérisé en ce que**
le gel de silicone disposé dans la partie inférieure (22) présente une force de cohésion comprise entre 23 et 40 N, dans lequel la force de cohésion est une valeur obtenue en mesurant la pression au moment où le gel de silicone libéré à partir d'un orifice circulaire de 1 mm est séparé lorsqu'une pression négative est appliquée; et
la partie inférieure occupe 20 à 50 % en volume par rapport au volume total de l'intérieur de l'enveloppe en silicone (21).
